(19) Europäisches Patentamt
European Patent Office
Office européen des brevets

(11) Publication number: 0 017 264
B1

(12) **EUROPEAN PATENT SPECIFICATION**

(45) Date of publication of patent specification: 02.02.83

(51) Int. Cl.³: C 07 D 221/04
//C07D209/52

(21) Application number: 80200133.9

(22) Date of filing: 15.02.80

# (54) Bicyclic lactams and a process for their preparation.

(30) Priority: 23.02.79 GB 7906412

(43) Date of publication of application:
15.10.80 Bulletin 80/21

(45) Publication of the grant of the patent:
02.02.83 Bulletin 83/5

(84) Designated Contracting States:
BE CH DE FR GB IT NL SE

(56) References cited:
CHEMICAL ABSTRACTS, 9th Collective Index, Chemical Substance, 1978, page 2468CS Columbus, Ohio, U.S.A.

(73) Proprietor: SHELL INTERNATIONALE RESEARCH MAATSCHAPPIJ B.V.
Carel van Bylandtlaan 30
NL-2596 HR Den Haag (NL)

(72) Inventor: Scholes, Gary
Badhuisweg 3
NL-1031 CM Amsterdam (NL)
Inventor: Baardman, Frank
Badhuisweg 3
NL-1031 CM Amsterdam (NL)

(74) Representative: Rogers, Roy Charles et al,
4 York Road
London SE1 7NA (GB)

EP 0 017 264 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Bicyclic lactams and a process for their preparation

The present invention relates to certain bicyclic lactams which are useful intermediates in the preparation of biologically active compounds, and to a process for their preparation.

The invention provides a compound of the general formula:

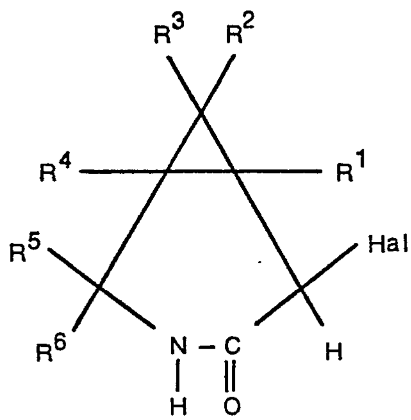

(I)

wherein each of $R^1$, $R^2$ $R^3$, $R^4$, $R^5$ and $R^6$, any two or more of which may be the same or different, represents a hydrogen atom, a carboxy group, or an alkyl group which may be unsubstituted or substituted by one or more alkoxy groups or $R^1$ and $R^4$ have the above meaning and $R^2$ and $R^3$ and/or $R^5$ and $R^6$ together represent an alkylene group, and Hal represents a chlorine, bromine or iodine atom.

Preferably each of $R^1$, $R^2$, $R^3$, $R^4$, $R^5$ and $R^6$, represents a hydrogen atom, an alkyl group having up to six carbon atoms and Hal represents a chlorine atom. An especially preferred compound is 5-chloro-3-azabicyclo[4.1.0]heptan-4-one- i.e. the compound of formula I in which each of $R^1$ to $R^6$ represents a hydrogen atom, and Hal represents a chlorine atom.

It will be appreciated that the compounds according to the general formula I exhibit both optical and geometric isomerism. Geometric isomerism with respect to the halogen moiety is of the cis/trans nature reflecting the mutual positions of the halogen moiety and the $CR^2R^3$ bridging group.

The invention also provides a process for the preparation of a compound of the general formula I, characterised in that an N-substituted bicyclic lactam of the general formula

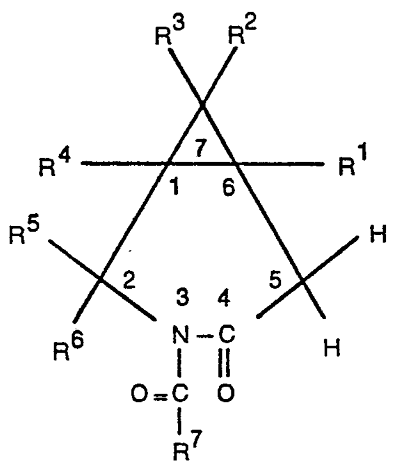

(II)[a]

wherein $R^1$, $R^2$, $R^3$, $R^4$, $R^5$ and $R^6$ have the meanings defined hereinabove and $R^7$ represents an unsubstituted or substituted alkyl, aryl, alkoxy or aryloxy group, is reacted with a halogenating agent, and the 5-monohalo compound obtained is converted into the bicyclic lactam of general formula I.

$R^7$ may for example represent a group having up to 10 carbon atoms. Preferably $R^7$ represents a secondary or tertiary alkyl group, a phenyl group, or an alkoxy group having up to 6 carbon atoms. Most preferably $R^7$ represents a phenyl group.

Thus an especially preferred embodiment of the process according to the present invention is one in which 5-chloro-3-azabicyclo[4.1.0]heptan-4-one is prepared from 3-benzoyl-3-azabicyclo[4.1.0]-heptan-4-one.

The halogenating agent to be used in the process according to the present invention can be any conventional halogenating agent. Suitable chlorinating agents include sulphuryl chloride, phosphorus

pentachloride, N-chlorosuccinimide, alkali metal hypochlorites, and molecular chlorine. Preference is given to the use of sulphuryl chloride or phosphorus pentachloride as chlorinating agent. Suitable brominating agents include sulphuryl bromide, phosphorus tribromide, bromine, N-bromo-succinimide, and alkali metal hypobromites. Preference is given to the use of sulphuryl bromide. A mixture of phosphorus and iodine can be used to introduce an iodine moiety at the 5-position of the bicyclic lactam of formula $II^a$.

The halogenation can be carried out conveniently at temperatures up to 150°C. For chlorination, temperatures between 50 and 80°C are preferred. Higher temperatures are normally required to introduce a bromine or iodine atom at the 5-position in the bicyclic lactam.

The presence of the group

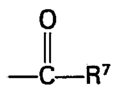

in the starting bicyclic lactam leads to the result that 5-monohalo compounds are selectively obtained in high yield.

The halogenation is preferably carried out in the presence of an inert solvent. Suitable solvents include alkanes, alkenes, cycloalkanes, and halogenated alkanes and alkenes, for example hexane, heptane, cyclopentane, cyclohexane, methylene dichloride, chloroform, carbon tetrachloride, dichloroethane, trichloroethylene and perchloroethylene, Also mixtures of solvents, e.g. a mixture of carbon tetrachloride and cyclohexane, can be used.

The halogenating agent can be applied in a stoichiometric amount with respect to the starting material according to formula $II^a$. Good results are normally obtained using a slight excess of halogenating agent, e.g. 5—20% mol, per mol of compound to be halogenated.

The 5-monohalo compound can be isolated from the reaction mixture by any conventional technique, e.g. by removal of the solvent or solvent mixture followed by one or more wash treatments and final drying. It is also possible and sometimes advantageous to use the crude product obtained in the halogenation step of the process according to the present invention *in situ* without purification.

The replacement of the protective acyl or ester group $COR^7$, by a hydrogen atom, can be carried out conveniently by treating the 5-monohalo compound either after isolation or as such in the crude reaction product with a strong acid. Suitable acids include mineral acids such as sulphuric acid and hydrochloric acid, and strong organic acids such as trifluoroacetic acid. Good results have been obtained using concentrated sulphuric acid. Usually the reaction will be carried out at ambient or somewhat elevated temperatures, e.g. temperatures up to 100°C.

The final product can be obtained by using conventional working-up techniques such as neutralising the reaction mixture followed by extraction with a suitable solvent. Evaporation of the solvent will then give the product which may further purified, e.g. by recrystallisation techniques if necessary.

The starting material of formula $II^a$ can be conveniently prepared from the corresponding 3-azabicyclo[4.1.0]heptan-4-one which can be obtained from the 3-hydroxyimino bicyclo[3.1.0]hexanes via a Beckmann rearrangement. A Beckmann rearrangement of some terpene oximes (cf. Chem. Abstr. 9th Collective Index, Chemical Substance, 1978, p. 2468CS) is disclosed in Chem. Abstr. Vol. 78, 1973, ref. 111524d.

Compounds of the general formula I are useful intermediates in the preparation of 3-azabicyclo-[3.1.0]hexane-2-carboxylic acid and certain derivatives thereof including salts and esters thereof, which have useful plant growth regulating and pollen suppressing activity (cf. British Patent Specification No. 1 567 153).

## Example 1

*Preparation of 5-chloro-3-azabicyclo[4.1.0]heptan-4-one*

(a) Preparation of 3-benzoyl-3-azabicyclo[4.1.0]heptan-4-one from 3-hydroxyimino-bicyclo[3.1.0]hexane.

To an ice-cold solution of 6.5 g (0.06 mole) of 3-hydroxyimino[3.1.0]hexane (c.f. J.Am.Chem.Soc., *85* [1963] 1782) in dry ether (40 ml) was added a solution of 13 ml thionylchloride (0.18 mole) in dry ether (40 ml) over a period of 30 minutes whilst stirring. A white solid separated which gradually turned brown. The stirring was continued at room temperature for 5 hours. After the removal of solvent and excess thionyl chloride under reduced pressure, ice was added to the brown residue. The aqueous solution formed was made alkaline by the addition of a dilute NaOH solution and then extracted with 100 ml portions (x5) of methylene dichloride. The combined extracts were dried over anhydrous magnesium sulphate and then boiled with Norit to remove most of the brown discolouration. Evaporation of the solvent then left a light brown residue which solidified on standing. The yield of 3-azabicyclo[4.1.0]heptan-4-one was 4.5 g (68%).

The compound was characterised by the following data:

N.M.R. ($CDCl_3$, chemical shift in ppm from tetramethylsilane as internal standard):

7.5 bs (1H,N—H); 3.7 d(1H,$H_2$); 3.45 dd (1H,$H_2$); 2.61 (2H,$H_5$); 1.20 m(2H.$H_1$,$H_6$); and 0.60 m (2H,$H_7$)

I.R. (neat; in $cm^{-1}$): 3250 m (N—H stretch); 1680 s (C=O stretch); 1500 s(N—H bend); 1350 s(C—O stretch); 1260 w; 1218 w; 1140 m; 1110 m; 1050 w; 1035 w; 1000 m(cyclopropyl); and 925 w.

To the product obtained were added 6.3 g benzoyl chloride (0.045 mole) and 5.4 g dimethylaniline (0.045 mole) and the mixture was stirred at 70°C for three hours. The reaction mixture was then allowed to cool and then poured onto an ice dilute hydrochloric acid mixture and extracted with methylene dichloride (x3). The collected organic layers were dried over anhydrous magnesium sulphate. The solvent was evaporated and last traces of excess benzoyl chloride were removed by pumping with a vacuum pump. The yield of 3-benzoyl-3-azabicyclo[4.1.0]heptan-4-one was 5 g (58% calculated on the starting material 3-azabicyclo[4.1.0]heptan-4-one.

The compounds was characterised by the following data:

N.M.R. ($CDCl_3$, chemical shift in ppm from tetramethyl silane as internal standard):

7.4 m (5H, phenyl); 4.1 dd (1H, $H_2$);
3.9 dd (1H, $H_2$); 2.7 t (2H, $H_5$); 1.3 m (2H, $H_1$ and $H_6$); and 1.0—0.5 m (2H, $H_7$).

(b) Preparation of 5-chloro-3-benzoyl-3-azabicyclo[4.1.0]heptan-4-one

A mixture of 5 g (0.023 mole) 3-benzoyl-3-azabicyclo[4.1.0]heptan-4-one and 3.5 g (0.026 mole) sulphuryl-chloride was heated under reflux for two hours in a mixture of carbon tetrachloride (1 ml) and cyclohexane (3 ml) as solvent. After removal of the solvent mixture by evaporation, absolute ethanol (10 ml) was added and removed by evaporation one hour later to obtain a dry product which was not purified further. The yield of 5-chloro-3-benzoyl-3-azabicyclo[4.1.0]heptan-4-one was 5 g (87% calculated as starting material 3-benzoyl-3-azabicyclo[4.1.0]heptan-4-one).

The compound was characterised by the following data:

N.M.R. ($CDCl_3$, chemical shift in ppm from tetramethylsilane as internal standard);

7.5 m (5H, phenyl); 4.6 bs (1H, $H_5$); 4.0 d(1H, $H_2$); 3.7 d (1H, $H_2$); 1.8—1.2 m (2H, $H_1$ and $H_6$); and 0.9—0.4 m (2H, $H_7$).

(c) Preparation of 5-chloro-3-azabicyclo[4.1.0]heptan-4-one

The benzoylchlorolactam obtained in experiment (b) (5 g (0.02 mole)) was added to 96% sulphuric acid (10 ml) and stirred for three hours at 80°C. After cooling, the reaction mixture was neutralised with ammonium hydroxide and extracted with methylene chloride (x3). After evaporation of the solvent the residue was purified by passing over a column of neutral alumina and eluting with diethylether to give 5-chloro-3-azabicyclo[4.1.0]heptan-4-one as a yellow solid. Recrystallisation from diethyl ether/pentane gave 2 g of pale yellow crystals of 5-chloro-3-azabicyclo[4.1.0]heptan-4-one. The yield was 60% (calculated on the starting benzoylchlorolactam).

The compound was characterised by the following data:

N.M.R. ($CDCl_3$, chemical shift in ppm from tetramethylsilane as internal standard):

6.6 bs (1H, N—H); 4.5 s (1H, $H_5$); 3.8 d (1H, $H_2$); 3.4 dd (1H, $H_2$); 1.8—1.1 m (2H, $H_1$ and $H_6$) and 0.7 m (2H, $H_7$).

The compound obtained was characterised as the trans chloro compound on the basis of the $H_5$—$H_6$ coupling constant ($J_{5,6}$ = 2.5 Hz).

I.R. (neat in $cm^{-1}$) = 3300, 1690, 735.

## Example 2

*Preparation of 2-carboxy-3-azabicyclo[3.1.0]hexane*

0.9 g of the chlorolactam prepared in Example 1 were refluxed for 16 hours in t-butanol containing 0.5 g potassium. The reaction mixture was allowed to cool, the solvent was evaporated under reduced pressure and the residue poured into 20 ml $H_2O$. Dilute (4N) HCl was added until the solution was acidic and then extracted (x3) with methylene chloride. The water layer was then passed over a column filled with Dowex 50W—X8 ion-exchange resin (DOWEX is a Trade Mark). Eluting with ammonium hydroxide then gave 120 mg of the cis isomer of 2-carboxy-3-azabicyclo[3.1.0]hexane.

**Claims**

1. A compound of the general formula:

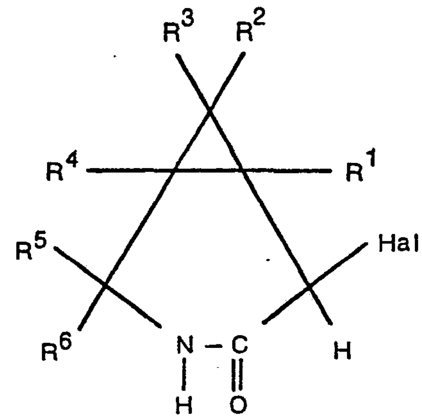

(I)

wherein each of $R^1$, $R^2$, $R^3$, $R^4$, $R^5$ and $R^6$, any two or more of which may be the same or different, represents a hydrogen atom, a carboxy group, or an alkyl group which may be unsubstituted or substituted by one or more alkoxy groups or $R^1$ and $R^4$ have the above meaning and $R^2$ and $R^3$ and/or $R^5$ and $R^6$ together represent an alkylene group, and Hal represents a chlorine, bromine or iodine atom.

2. A compound as claimed in claim 1, in which each of $R^1$, $R^2$, $R^3$, $R^4$, $R^5$ and $R^6$, any two or more of which may be the same or different, represents a hydrogen atom or an alkyl group having up to 6 carbon atoms, and Hal represents a chlorine atom.

3. 5-Chloro-3-azabicyclo[4.1.0]heptan-4-one.

4. A process for the preparation of a compound as claimed in any one of claims 1 to 3, characterised in that an N-substituted bicyclic lactam of the general formula:—

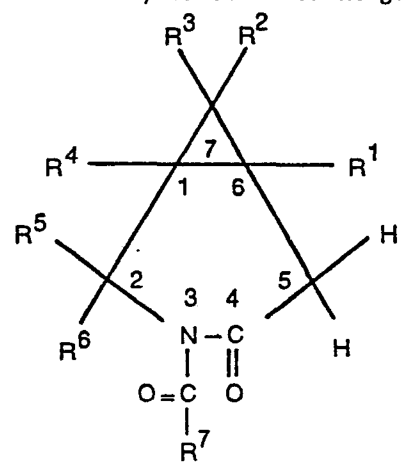

(II)[a]

wherein $R^1$, $R^2$, $R^3$, $R^4$, $R^5$ and $R^6$ have the meanings defined in claim 1 and $R^7$ represents an unsubstituted or substituted alkyl, aryl, alkoxy or aryloxy group, is reacted with a halogenating agent under formation of a 5-monohalo compound of the general formula:—

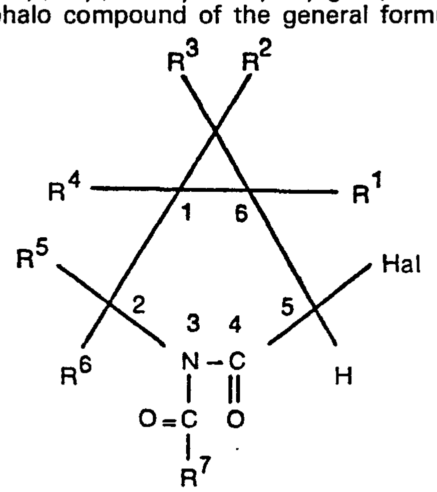

(II)[b]

wherein $R^1$, $R^2$, $R^3$, $R^4$, $R^5$, $R^6$ and $R^7$ have the meanings defined hereinabove and the 5-monohalo-compound obtained is converted by treatment with a strong acid into the bicyclic lactam of general formula I.

5. A process as claimed in claim 4, characterised in that $R^7$ represents an alkoxy group having up to 6 carbon atoms, a secondary or tertiary alkyl group, or a phenyl group.

6. A process as claimed in claim 5, characterised in that $R^7$ represents a phenyl group.

7. A process as claimed in any one of claims 4 to 6 characterised in that the halogenating agent is sulphuryl chloride, phosphorus pentachloride, or sulphuryl bromide.

8. A process as claimed in any one of claims 4 to 7 characterised in that the 5-monohalo compound prepared is converted into a compound of the general formula I by treatment with a mineral acid.

9. A process as claimed in claim 8, characterised in that the acid is sulphuric acid.

**Revendications**

1. Un composé de la formule générale:

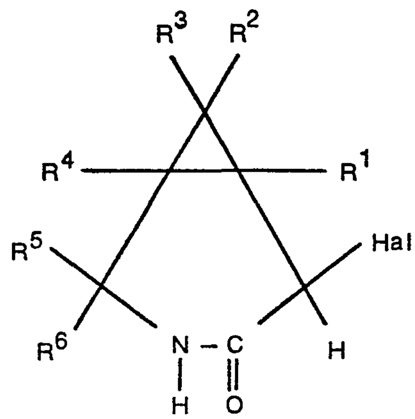

(I)

dans laquelle $R^1$, $R^2$, $R^3$, $R^4$, $R^5$ et $R^6$, dont deux quelconques ou plus peuvent être identiques ou qui peuvent être tous différents, représentent chacun un atome d'hydrogène, un groupe carboxy ou un groupe alcoyle qui peut être non-substitué ou substitué par un ou plusieurs groupes alcoxy ou $R^1$ et $R^4$ ont la signification ci-dessus et $R^2$ et $R^3$ et/ou $R^5$ et $R^6$ représentent ensemble un groupe alcoylène et Hal représente un atome de chlore, de brome ou d'iode.

2. Un composé selon la revendication 1, dans lequel $R^1$, $R^2$, $R^3$, $R^4$, $R^5$ et $R^6$ dont deux quelconques ou plus peuvent être identiques ou qui peuvent être tous différents, représentent chacun un atome d'halogène ou un groupe alcoyle ayant jusqu'à 6 atomes de carbone et Hal représente un atome de chlore.

3. 5-chloro-3-azabicyclo[4.1.0]heptan-4-one.

4. Un procédé pour la préparation d'un composé tel que revendiqué dans l'une quelconque des revendications 1 à 3, caractérisé en ce qu'un lactame bicyclique N-substitué de la formule générale:

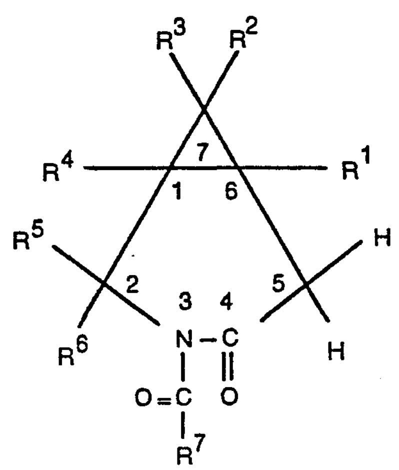

(II)a

dans laquelle $R^1$, $R^2$, $R^3$, $R^4$, $R^5$ et $R^6$ ont les significations définies dans la revendication 1 et $R^7$ représente une groupe alcoyle, aryle, alcoxy ou aryloxy non substitué ou substitué, est mis à réagir avec un agent d'halogénation pour formation d'un composé 5-monohalogéno de la formule générale:

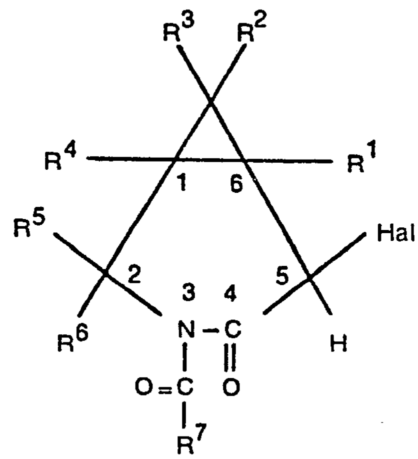

(II)[b]

dans laquelle $R^1$, $R^2$, $R^3$, $R^4$, $R^5$, $R^6$ et $R^7$ ont les significations définies ci-dessus et le composé 5-monohalogéno obtenu est transformé par un traitement par un acide fort en lactame bicyclique de la formule générale I.

5. Un procédé selon la revendication 4, caractérisé en ce que $R^7$ représente un groupe alcoxy ayant jusqu'à 6 atomes de carbone, un groupe alcoyle secondaire ou tertiaire ou un groupe phényle.

6. Un procédé selon la revendication 5, caractérisé en ce que $R^7$ représente un groupe phényle.

7. Un procédé selon l'une quelconque des revendications 1 à 6, caractérisé en ce que l'agent d'halogénation est du chlorure de sulfuryle, du pentachlorure de phosphore ou du bromure de sulfuryle.

8. Un procédé selon l'une quelconque des revendications 4 à 7, caractérisé en ce que le composé 5-monohalogéno préparé est transformé en un composé de la formule générale I par traitement par un acide minéral.

9. Un procédé selon la revendication 8, caractérisé en ce que l'acide est de l'acide sulfurique.

**Patentansprüche**

1. Verbindung der allgemeinen Formel

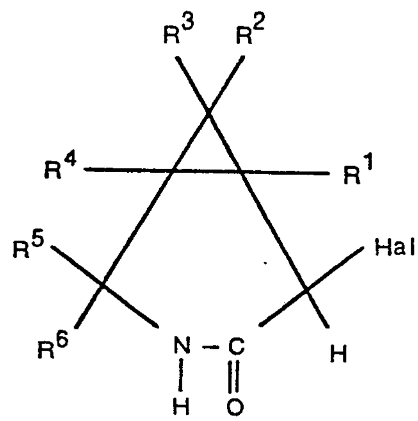

(I)

in der $R^1$, $R^2$, $R^3$, $R^4$, $R^5$ und $R^6$, von denen jeweils zwei oder mehrere gleich oder verschieden sein können, jeweils ein Wasserstoffatom, eine Carboxylgruppe oder eine Alkylgruppe die unsubstituiert oder durch ein oder mehrere Alkylgruppen substituiert sein kann, bedeuten oder $R^1$ und $R^4$ die oben angegebene Bedeutung haben und $R^2$ und $R^3$ und/oder $R^5$ und $R^6$ zusammen eine Alkylengruppe bedeuten und Hal ein Chlor-, Brom- oder Jodatom ist.

2. Verbindung nach Anspruch 1, in der $R^1$, $R^2$, $R^3$, $R^4$, $R^5$ und $R^6$, von denen jeweils zwei oder mehrere gleich oder verschieden sein können, jeweils ein Wasserstoffatom oder eine Alkylgruppe mit bis zu 6 Kohlenstoffatomen und Hal ein Chloratom bedeuten.

3. 5-Chlor-3-azabicyclo[4.1.0]heptan-4-on.

4. Verfahren zur Herstellung einer Verbindung nach einem der Ansprüche 1 bis 3, dadurch gekennzeichnet, daß ein N-substituiertes bicyclisches Lactam der allgemeinen Formel

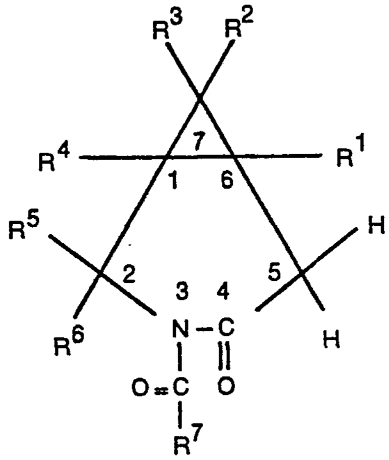

(II)[a]

in der $R^1$, $R^2$, $R^3$, $R^4$, $R^5$ und $R^6$, die in Anspruch 1 angegebene Bedeutung haben und $R^7$ eine unsubstituierte oder substituierte Alkyl-, Aryl-, Alkoxy- oder Aryloxygruppe ist, umgesetzt wird mit einem Halogenierungsmittel unter Bildung einer 5-Mono-halogenverbindung der allgemeinen Formel

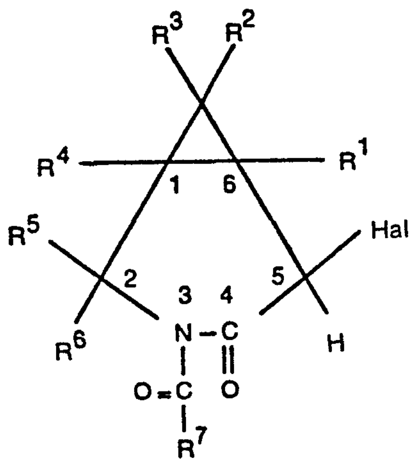

(II)[b]

in der $R^1$, $R^2$, $R^3$, $R^4$, $R^5$, $R^6$ und $R^7$ die oben angegebene Bedeutung haben, und die erhaltene 5-Monohalogenverbindung durch Behandlung mit einer starken Säure in das bicyclische Lactam der allgemeinen Formel I umgewandelt wird.

5. Verfahren nach Anspruch 4, dadurch gekennzeichnet, daß $R^7$ eine Alkoxygruppe mit bis zu 6 Kohlenstoffatomen, eine sekundäre oder tertiäre Alkylgruppe oder eine Phenylgruppe bedeutet.

6. Verfahren nach Anspruch 5, dadurch gekennzeichnet, daß $R^7$ eine Phenylgruppe bedeutet.

7. Verfahren nach einem der Ansprüche 4 bis 6, dadurch gekennzeichnet, daß das Halogenierungsmittel Sulfurylchlorid, Phosphorpentachlorid oder Sulfurylbromid ist.

8. Verfahren nach einem der Ansprüche 4 bis 7, dadurch gekennzeichnet, daß die hergestellte 5-Monohalogenverbindung durch Behandlung mit einer Mineralsäure in eine Verbindung der allgemeinen Formel I umgewandelt wird.

9. Verfahren nach Anspruch 8, dadurch gekennzeichnet, daß die Säure Schwefelsäure ist.